Berichtigte Neuausgabe (dat. . . Dez.83. . . .) (des) (der)

[ ] gesamten
[X] Titelblattes
[ ] Seite(n)

[X] (der) Patentanmeldung . 0.091.749 A2
Dat.(43) .19.10.83. . . . .
[ ] (der) Patentschrift . . . . . . . . . . . . . . .
Dat.(45) . . . . . . . . . . . . . .

Die Berichtigung bezieht sich auf: (57)

Amended edition (dated . . dec.83 . . . . .) of

[ ] entire
[X] title page
[ ] page(s)

[X] (of) patent application . 0.091.749. A2 .
dat.(43) . .19.10.83. . . . .
[ ] (of) patent specification . . . . . . . . . . . . . . .
dat.(45) . . . . . . . . . . . . . . .

The amendment refers to: (57)

Nouvelle edition corrigée (datée du .dec.83. . . . . .) de

[ ] la totalité
[X] la page de garde
[ ] la (les) page(s)

[X] de la demande 0·091·749·A2· · ·
dat.(43) 19.10.83. . . . . . .
[ ] du fascicule de brevet . . . . . . . . . . . . . . . .
dat.(45) . . . . . . . . . . . . . . . .

La correction porte sur : (57)

EPA - EPO - OEB
DG 1
Reçu le
05 JAN 1984

Europäisches Patentamt
European Patent Office
Office européen des brevets

(19)

(11) Publication number: 0 091 749 A2

(12) EUROPEAN PATENT APPLICATION

(21) Application number: 83301652.0

(22) Date of filing: 24.03.83

(51) Int. Cl.³: C 07 C 143/78
C 07 C 143/79, C 07 D 295/22
A 61 K 31/18, A 61 K 31/445
A 61 K 31/535

(30) Priority: 08.04.82 GB 8210492

(43) Date of publication of application: 19.10.83 Bulletin 83/42

(84) Designated Contracting States: CH DE FR GB IT LI NL

(71) Applicant: BEECHAM GROUP PLC
Beecham House Great West Road
Brentford Middlesex(GB)

(72) Inventor: Duckworth, David Malcolm
44 Meadow Walk
Walton on the Hill Tadworth Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Ethanolamine derivatives, process for their preparation and pharmaceutical compositions containing them.**

(57) A compound of formula (I):

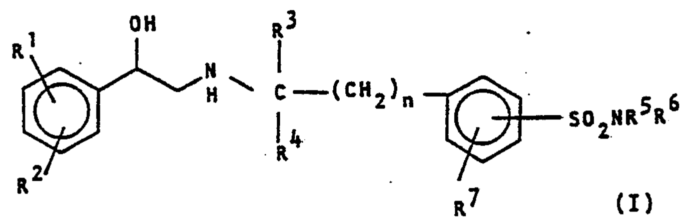

(I)

or a salt thereof, wherein;
$R^1$ is hydrogen, halogen or trifluoromethyl,
$R^2$ is hydrogen or halogen,
$R^3$ is hydrogen or methyl,
$R^4$ is hydrogen or methyl,
$R^5$ is hydrogen, $C_{1-10}$ straight or branched alkyl or optionally substituted benzyl,
$R^6$ is hydrogen, $C_{1-10}$ straight or branched alkyl, or optionally substituted benzyl,
or $R^5$ and $R^6$ taken together with the attached nitrogen atom represent a heterocyclic ring,
$R^7$ is hydrogen or halogen, and
n is 1 or 2;
are useful in the treatment of obesity and/or diabetes.

EP 0 091 749 A2

Croydon Printing Company Ltd.

(19) Europäisches Patentamt
European Patent Office
Office européen des brevets

(11) Publication number: **0 091 749**
**A2**

(12) # EUROPEAN PATENT APPLICATION

(21) Application number: 83301652.0

(22) Date of filing: 24.03.83

(51) Int. Cl.³: C 07 C 143/78
C 07 C 143/79, C 07 D 295/22
A 61 K 31/18, A 61 K 31/445
A 61 K 31/535

(30) Priority: 08.04.82 GB 8210492

(43) Date of publication of application: 19.10.83 Bulletin 83/42

(84) Designated Contracting States: CH DE FR GB IT LI NL

(71) Applicant: BEECHAM GROUP PLC
Beecham House Great West Road
Brentford Middlesex(GB)

(72) Inventor: Duckworth, David Malcolm
44 Meadow Walk
Walton on the Hill Tadworth Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Ethanolamine derivatives, process for their preparation and pharmaceutical compositions containing them.**

(57) Anti-hypertensive compounds of formula (I):

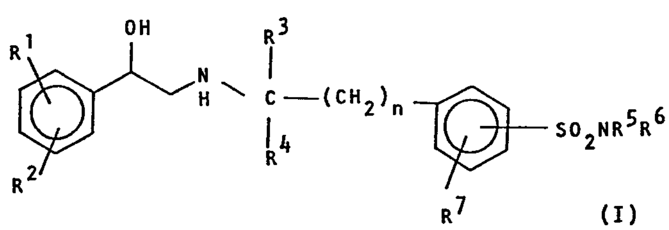

(I)

wherein

one of $R_1$ and $R_2$ is nitro, cyano or $C_{1-3}$ alkylcarbonyl, and the other is methoxy or amino optionally substituted by one or two $C_{1-6}$ alkyl or by $C_{2-7}$ alkanoyl;

one of $R_3$ and $R_4$ is hydrogen or $C_{1-4}$ alkyl and the other is $C_{1-4}$ alkyl, or $R_3$ and $R_4$ together with the carbon atom to which they are attached are $C_{3-6}$ spiroalkyl;

$R_5$ is hydrogen, $C_{1-3}$ alkyl or $C_{1-8}$ acyl; and

n is 1 or 2; the lactam group being trans to the $OR_5$ group; or, when the other of $R_1$ and $R_2$ is amino, a pharmaceutically acceptable salt thereof.

EP 0 091 749 A2

Ethanolamine derivatives, process for their preparation and pharmaceutical compositions containing them

The present invention relates to derivatives of ethanolamine which have anti-obesity and/or anti-hyperglycaemic activity, to processes for their production and to their use in medicine.

U.K. Patent Specification No. 1,301,134 discloses compounds of the general formula (A):

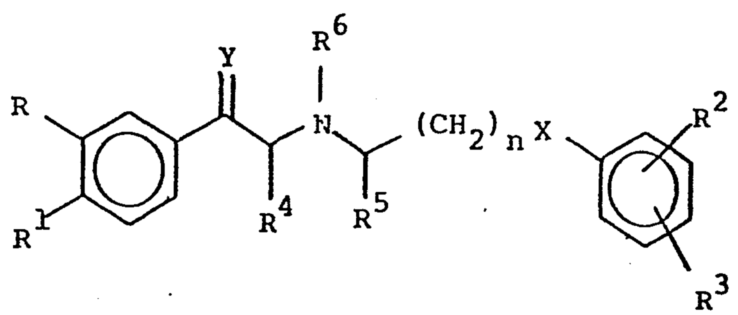

(A)

where R and $R^1$ each represent hydrogen or a hydroxy group, at least one being a hydroxyl group; $R^2$ represents hydrogen, halogen or a lower alkyl or alkoxy group; $R^3$ represents an acylamino group, a lower alkoxycarbonylamino group, an amoyl group or a ureido group, any one of which may be separated from the phenyl ring by a methylene or ethylene group; $R^4$, $R^5$ and $R^6$ each represent hydrogen or a lower alkyl group; X represents oxygen, sulphur, imino or a direct link;

Y represents a hydrogen atom and a hydroxy group, two hydrogen atoms or an oxygen atom; and n is 1, 2 or 3 when X is other than a direct link and is 0 to 4 when X is a direct link.

These compounds are said be be β-agonists and capable of increasing the force of the myocardial contraction. Furthermore, they are said to be useful in the curative or prophylactic treatment of cardiac conditions such as congestive heart failure, in the treatment of obstructive airways disease, and in the treatment of peripheral vascular disease.

We have now found a class of novel ethanolamine derivatives having some structural similarities to the compounds of formula (A), but which have anti-obesity and/or anti-hyperglycaemic activity coupled with low cardiac stimulant activity for particular members of the class.

Accordingly, the present invention provides a compound of formula (I):

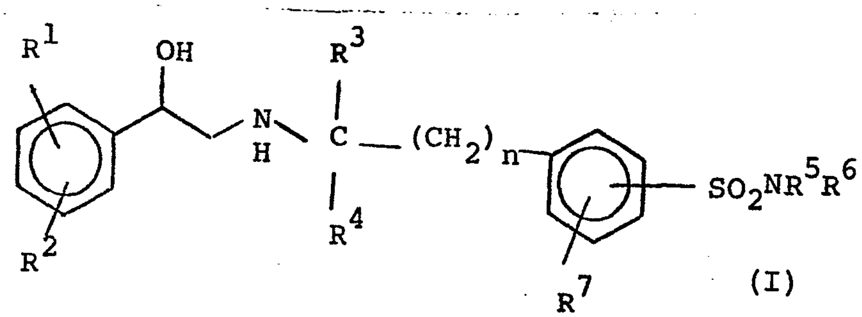

(I)

or a salt thereof, wherein;

$R^1$ is hydrogen, halogen or trifluoromethyl,

$R^2$ is hydrogen or halogen,

$R^3$ is hydrogen or methyl,

$R^4$ is hydrogen or methyl,

$R^5$ is hydrogen, $C_{1-10}$ straight or branched alkyl or optionally substituted benzyl,

$R^6$ is hydrogen, $C_{1-10}$ straight or branched alkyl, or optionally substituted benzyl,

or $R^5$ and $R^6$ taken together with the attached nitrogen atom represent a heterocylic ring,
$R^7$ is hydrogen or halogen, and
n is 1 or 2

Suitably, $R^3$ is hydrogen and $R^4$ is simultaneously methyl.

Suitably, $R^5$ is a $C_{1-5}$ straight chain alkyl group, preferably a methyl, n-propyl or n-butyl group, a benzyl group or a hydrogen atom.

Suitably, $R^6$ is a $C_{1-5}$ straight chain alkyl group, preferably a methyl, n-propyl or n-butyl group; a benzyl group or a hydrogen atom.

When $R^5$ and $R^6$ are taken together with the attached nitrogen atom they represent, suitably, a five or six membered heterocyclic ring, optionally containing an additional hetero atom selected from oxygen, sulphur or nitrogen. Preferred rings are piperidine or morpholine.

The following are examples of compounds of the present invention:

N-[2-(4-sulphonamidophenyl)-1-methylethyl]-2-hydroxy-2-(3-trifluoromethylphenyl)ethanamine;

N-[2-(4-N,N-dimethylsulphonamidophenyl)-1-methylethyl]-2-hydroxy-2-(3-trifluoromethylphenyl)ethanamine;

N-[2-(4-N,N-dimethylsulphonamidophenyl)-1-methylethyl]-2-hydroxy-2-(3-chlorophenyl)ethanamine;

N-[2-(4-N,N-di-n-propylsulphonamidophenyl)-1-methylethyl]-2-(3-chlorophenyl)-2-hydroxyethanamine;

N-[2-(4-N,N-di-n-propylsulphonamidophenyl)-1-methylethyl]-2-hydroxy-2-(3-trifluoromethylphenyl)ethanamine;

N-[2-(4-(1-piperidylsulphonyl)phenyl)-1-methylethyl]-2-hydroxy-2-(3-trifluoromethylphenyl)ethanamine;

N-[2-(4-N-n-butylsulphonamidophenyl)-1-methylethyl]-2-(3-chlorophenyl)-2-hydroxyethanamine;

N-[2-(4-N-benzylsulphonamidophenyl)-1-methylethyl]-2-(3-chlorophenyl)-2-hydroxyethanamine;

N-[2-(4-N-n-butylsulphonamidophenyl)-1-methylethyl]-2-hydroxy-2-(3-trifluoromethylphenyl)ethanamine;

N-[2-(4-N-methylsulphonamidophenyl)-1-methylethyl]-2-hydroxy-2-(3-trifluoromethylphenyl)ethanamine;

N-[2-(4-N,N-diisopropylsulphonamidophenyl)-1-methylethyl]-2-hydroxy-2-(3-trifluoromethylphenyl)ethanamine;

N-[2-(4-(4-morpholinosulphonyl)phenyl)-1-methylethyl]-2-hydroxy-2-(3-trifluoromethylphenyl)ethanamine;

N-[2-(4-N,N-diethylsulphonamidophenyl)-1-methylethyl]-2-hydroxy-2-(3-trifluoromethylphenyl)ethanamine; and

N-[2-(3-N,N-diethylsulphonamidophenyl)-1-methylethyl]-2-hydroxy-2-(trifluoromethylphenyl) ethanamine.

Pharmaceutically acceptable salts of compounds of formula (I) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid,

fumaric acid, malic acid, succinic acid, salicylic acid or acetylsalicyclic acid.

The salts of compounds of formula (I) need not be pharmaceutically acceptable as they are also useful in the preparation of other compounds of formula (I) and in the separation of stereoisomers of compounds of formula (I) when the salt ion is also optically active.

Compounds of formula (I) have at least one asymmetric carbon atom, i.e. the carbon atom bearing the hydroxyl and substituted phenyl groups, and, a second asymmetric carbon atom exists when $R^3$ and $R^4$ are different. The compounds may, therefore, exist in at least two and often four stereoisomeric forms. The present invention encompasses all stereoisomers of the compounds of formula (I) whether free from other stereoisomers or admixed with other stereoisomers in any proportion and thus includes, for instance, racemic mixtures of enantiomers.

Preferably, the carbon atom bearing the hydroxyl and substituted phenyl groups has the *R*-configuration.

The most potent compounds of formula (I) are those wherein $R^3$ and $R^4$ are different and both asymmetric carbon atoms have the *R*-configuration.

The absolute configuration of any compound of formula (I) may be determined by conventional X-ray crystallographic techniques.

It is believed that, in the $^{13}C$ nmr spectrum of compounds of formula (I) wherein one of $R^3$ and $R^4$ is hydrogen and the other is methyl, the diastereoisomer having the greater anti-obesity and anti-hyperglycaemic activity is that for which the signal of the methyl

group carbon atom appears at higher field (the lower numerical value when expressed in ppm) in $D_6$ dimethyl sulphoxide solution. The paired resonances often appear at approximately 20 ppm (less active) and slightly below 20 ppm (more active) down field from tetramethylsilane. Other paired resonances can occur for the carbon atoms attached directly to the nitrogen atom and the carbon which carries the hydroxyl and phenyl groups. Again the paired resonances of the more active diastereoisomer of the investigated compounds appear at the higher field position.

The present invention provides a process for producing a compound of formula (I), or a salt thereof, which process comprises reducing an oxo-group and/or double bond and/or cleaving a benzyl group of a compound of formula (II):

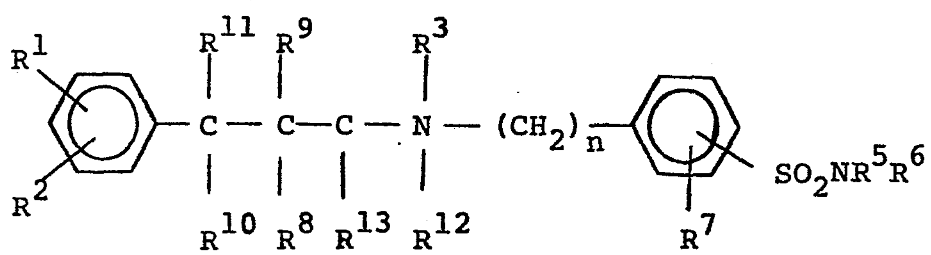

(II)

wherein $R^1$, $R^2$, $R^3$, $R^5$, $R^6$, $R^7$ and n are as defined in relation to formula (I),

$R^8$ is hydrogen or together with $R^{13}$ forms an oxo-group or together with $R^7$ forms a bond,

$R^9$ is hydrogen or together with $R^8$ forms an oxo-group,

$R^{10}$ is hydroxyl or together with $R^{11}$ forms an oxo-group,

$R^{11}$ is hydrogen or together with $R^{10}$ forms an oxo-group,

$R^{12}$ is a group $R^4$ as defined in relation to formula (I) or together with $R^{13}$ forms a bond,

$R^{13}$ is hydrogen or benzyl or together with $R^{12}$ or $R^8$ forms a bond,

provided that there is no more than one oxo-group represented by any of $R^8$ to $R^{11}$;and optionally thereafter forming a salt of the compound of formula (I) so formed.

Where there are two or more reducible moieties in the compound of formula (II) these may be reduced separately in any order or simultaneously.

The aforementioned reductions may be effected by conventional chemical methods or by catalytic methods. Suitably, chemical reduction may be effected with lithium aluminium hydride, sodium cyanoborohydride or sodium borohydride. Catalytic hydrogenation may be carried out using catalysts such as palladium on charcoal, or platinum, for instance, as platinum oxide.

Reduction by sodium borohydride is conveniently effected in a lower alkanolic solvent such as methanol or ethanol. The reaction is generally carried out at from 0-20°C.

Reduction by lithium aluminium hydride is conveniently effected in a dry, ether solvent such as diethyl ether or tetrahydrofuran at ambient or elevated temperatures.

Catalytic reduction is conveniently effected in a conventional hydrogenation solvent such as a lower alkanol, for instance ethanol. The hydrogenation is generally carried out under hydrogen gas at about 1 to 10 atmospheres pressure and at ambient or elevated temperatures.

Reduction of a compound of formula (II) wherein $R^{13}$ is benzyl is conveniently effected by catalytic

hydrogenation, preferably using palladium on charcoal as catalyst.

Preferred aspects of the process comprise reducing a compound of formula (IIA):

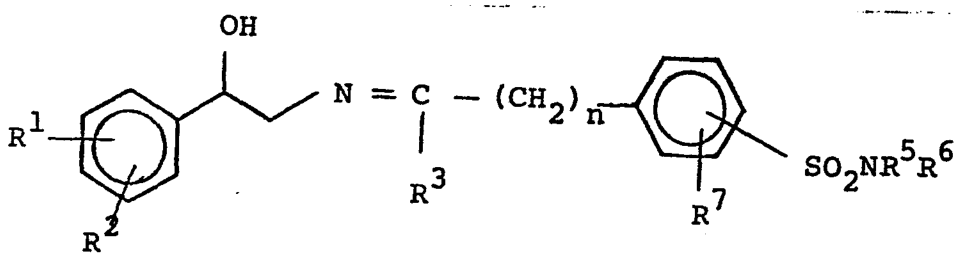

(IIA)

or reducing a compound of formula (IIB):

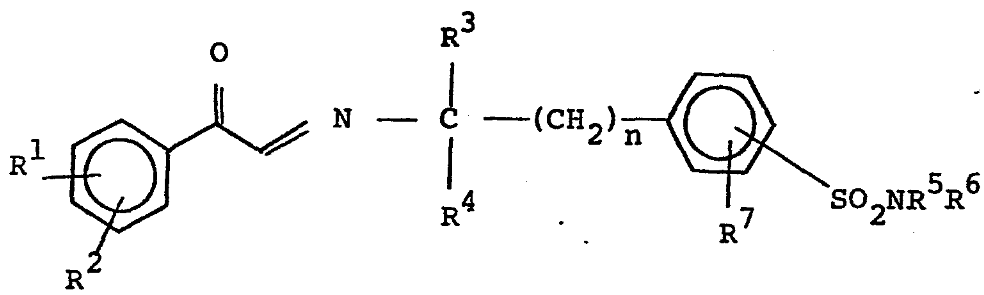

(IIB)

or reducing a compound of formula (IIC):

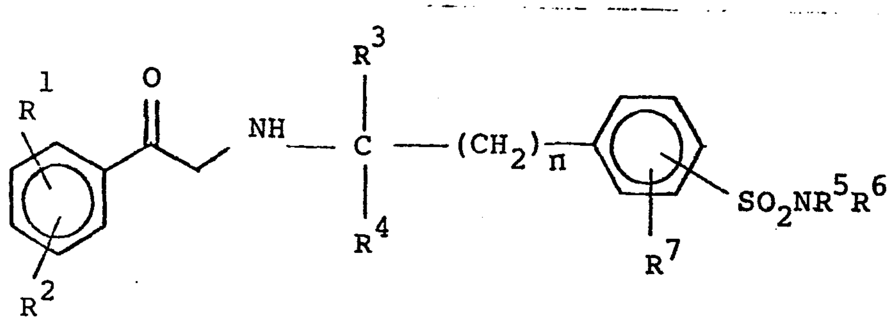

(IIC)

or reducing a compound of formula (IID)

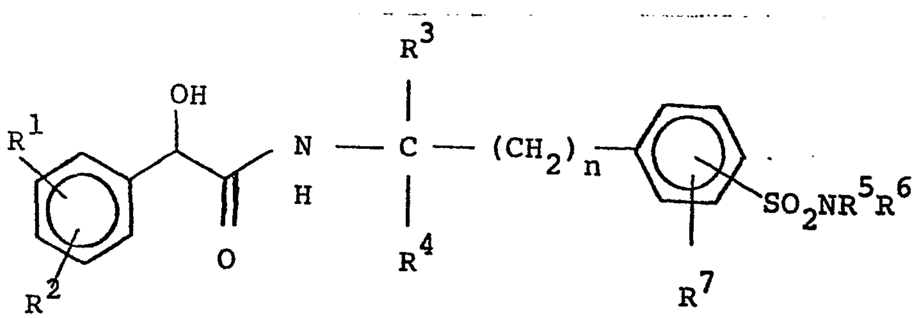

(IID)

or reducing a compound of formula (IIE):

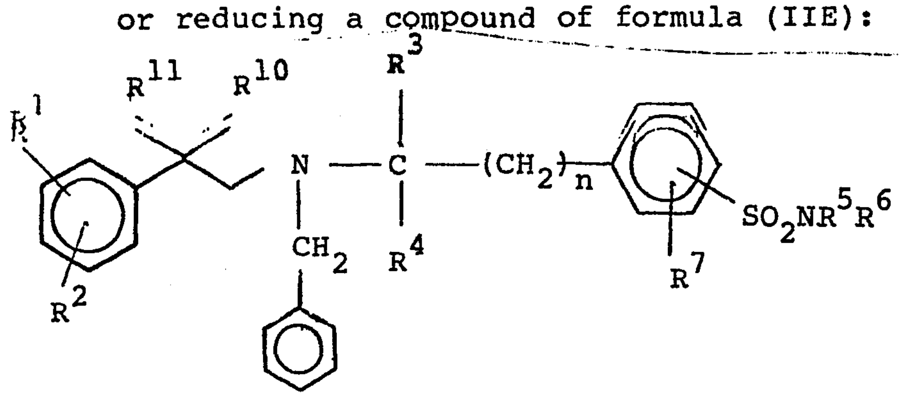

(IIE)

wherein $R^1$, $R^2$, $R^3$, $R^4$, $R^5$, $R^6$, $R^7$ and n are as defined in relation to formula (I) and $R^{10}$ and $R^{11}$ are as defined in relation to formula (II).

The present invention also provides another process for producing a compound of formula (I) or a salt thereof, which process comprises reacting a compound of formula (III):

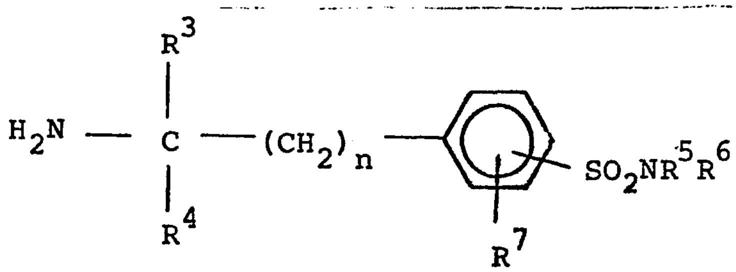

(III)

wherein $R^3$, $R^4$, $R^5$, $R^6$, $R^7$ and n are as defined in relation to formula (I);

with a compound of formula (IV):

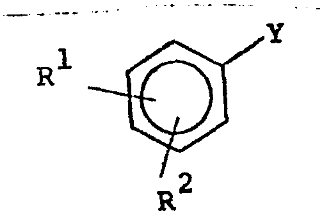

(IV)

wherein $R^1$ and $R^2$ are as defined in relation to formula (I), and Y is a group capable of reacting with the amine of formula (III) thus forming a compound of

formula (I), and optionally thereafter forming a salt of the compound of formula (I) so formed.

Typical examples of compounds of formula (IV) are compounds of formulae (IVA) and (IVB):

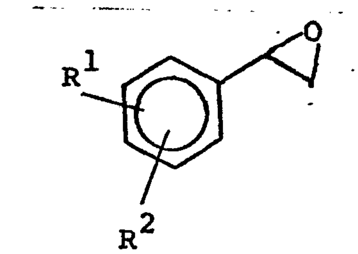

(IVA)

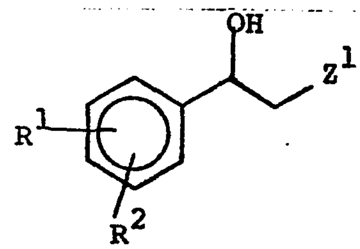

(IVB)

wherein $R^1$ and $R^2$ are as defined in relation to formula (I) and $Z^1$ is a leaving group, preferably halogen or tosyloxy.

The reaction of a compound of formula (III) with a compound of formula (IVA) is conveniently effected in a solvent such as a lower alkanol, preferably ethanol.

The reaction of a compound of formula (III) with a compound of formula (IVB) is conveniently conducted in a solvent, such as dimethyl sulphoxide, at elevated temperature, preferably about 50°C, for about 3 days.

The present invention provides a further process for the production of compounds of formula (I) or salts

thereof, which process comprises reacting a compound of formula (V):

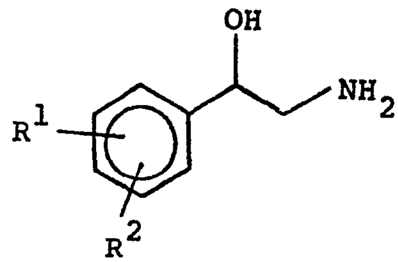

(V)

wherein $R^1$ and $R^2$ are as defined in relation to formula (I);

with a compound of formula (VI):

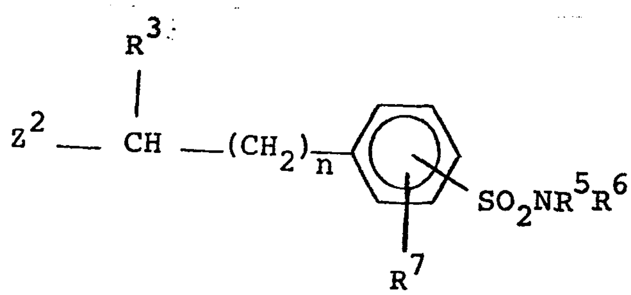

(VI)

wherein $R^3$, $R^5$, $R^6$, $R^7$ and n are as defined in relation to formula (I) and $Z^2$ is a leaving group preferably halogen or tosyloxy. The preparation of compounds of formula (V) is described in Published European Application No. 0 021 636.

The reaction of a compound of formula (V) with a compound of formula (VI) is conveniently effected in a solvent such as dimethylsulphoxide at an elevated temperature, preferably about 50°C for about two or three days.

A preferred process for producing compounds of formula (I) comprises the reduction of a compound of formula (IIA), especially using sodium borohydride in methanol at ambient temperatures.

The salts of compounds of formula (I) may be produced by treating the compound of formula (I) with the appropriate acid.

Compounds of formula (I) and salts thereof, produced by the above processes, may be recovered by conventional methods.

Compounds of formula (II) may be produced by reacting a compound of formula (III) as hereinbefore defined with a compound of formula (VII):

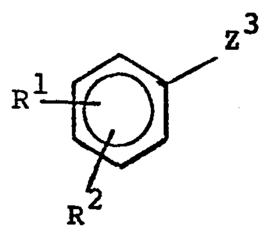

(VII)

wherein $R^1$ and $R^2$ are as defined in relation to formula (I) and $Z^3$ is a group which is capable of reacting with the amine of formula (III) thus forming a compound of formula (II). Typical examples of compounds of formula (VII) are:

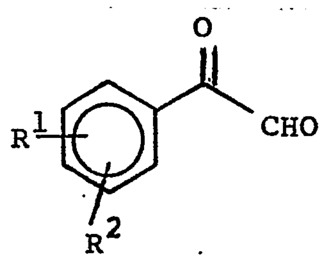

(VIIA)

or its hydrate or hemi-acetal of a lower alkanol;

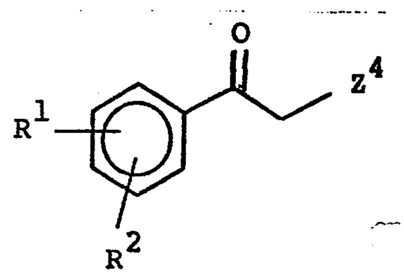

(VIIB)

0091749

wherein $Z^4$ is a leaving group, preferably bromine;

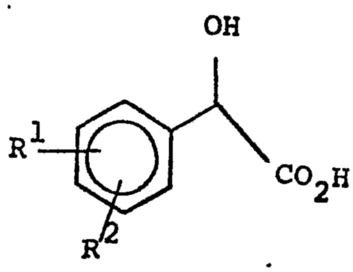

(VIIC)

wherein $R^1$ and $R^2$ are as defined in relation to formula (I).

Conventional conditions compatible with the particular compound of formula (VII) may be used for this reaction. Thus, the reaction of a compound of formula (VIIA) with a compound of formula (III) is conveniently conducted at elevated temperatures under conditions resulting in the removal of the water formed during the reaction. A particularly suitable method is to perform the reaction in a solvent, such as benzene, under reflux and to remove the water azeotropically using a Dean and Stark trap.

The reaction of a compound of formula (VIIB) with a compound of formula (III) is conveniently conducted in a polar organic solvent such as acetonitrile or butanone, at an elevated temperature, for instance under reflux.

The reaction of a compound of formula (VIIC) with a compound of formula (III) is conveniently conducted under standard peptide formation reaction conditions.

Alternatively a compound of formula (II) may be prepared by reacting a compound of formula (VIII):

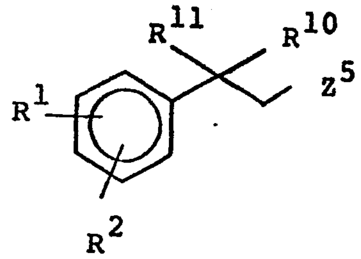

(VIII)

wherein $R^1$, $R^2$, $R^{10}$ and $R^{11}$ are as defined in relation to formula (II);

and $Z^5$ is a leaving group, preferably halogen or tosyloxy;

with a compound of formula (IX):

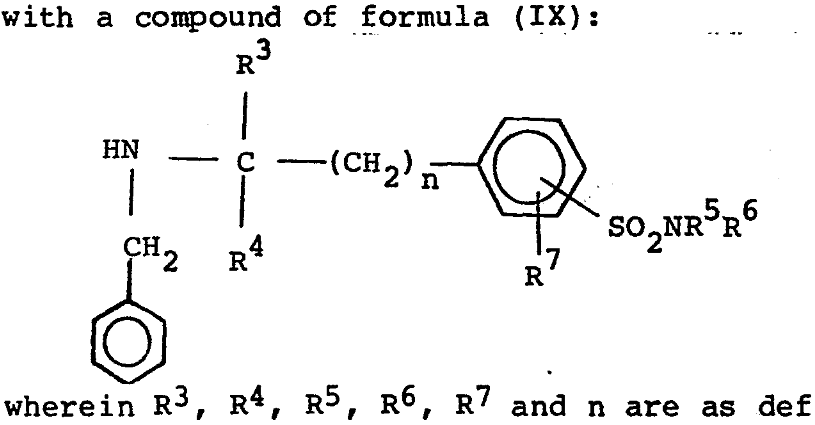

(IX)

wherein $R^3$, $R^4$, $R^5$, $R^6$, $R^7$ and n are as defined in relation to formula (II).

A particularly preferred process for producing certain compounds of formula (II) is reacting a compound of formula (V) as hereinbefore defined with a compound of the formula (X):

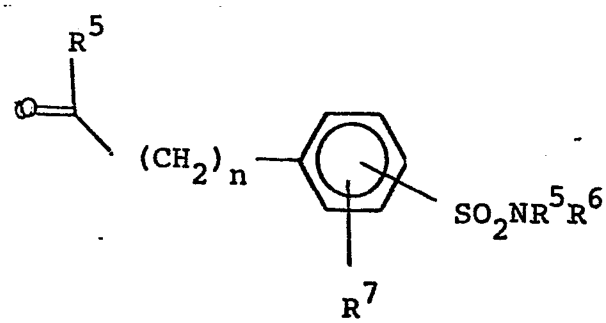

(X)

wherein $R^3$, $R^5$, $R^6$, $R^7$ and n are as defined in relation to formula (I).

The reaction of a compound of formula (V) with a compound of formula (X) is conveniently effected under conditions which result in the removal of water formed during the reaction. A particularly suitable method is to perform the reaction in a solvent, such as benzene, under reflux and to remove the water azeotropically using a Dean and Stark trap.

It is often convenient to prepare the compound of formula (II) and reduce it, in situ, to the desired compound of formula (I) without isolation of the compound of formula (II).

Compounds of formula (I) having a single asymmetric carbon atom may, if desired, be separated into individual enantiomers by conventional means, for example, by the use of an optically active acid as a resolving agent. Those compounds of formula (I) having two asymmetric carbon atoms may be separated into diastereoisomeric pairs of enantiomers by, for example, fractional crystallisation from a suitable solvent such as ethyl acetate. The pair of enantiomers thus obtained may be separated into individual stereoisomers by conventional means such as by the use of an optically active acid as a resolving agent.

Suitable optically active acids which may be used as resolving agents are described in "Topics in Stereochemistry", Vol. 6, Wiley Interscience, 1971, Allinger, N.L., and Eliel, W.L. Eds.

Alternatively any enantiomer of a compound of formula (I) may be obtained by stereospecific synthesis using optically pure starting materials of known configuration.

By using single enantiomers of a compound of formula (III) and of a compound of formula (VIIC) a

stereospecific synthesis of a compound of formula (II) is achieved. This may then be reduced to a compound of formula (I) without altering the configuration of the two asymmetric carbon atoms. Thus, for example, reaction of a compound of formula (III) with the R-absolute configuration and a compound of formula (VIIC) with the R-absolute configuration would afford a compound of formula (II) and, on reduction, a compound of formula (I) with the R,R-absolute configuration.

By reacting a single enantiomer of a compound of formula (III) with a single enantiomer of a compound of formula (IVA) or (IVB), the direct stereospecific synthesis of a single enantiomer of a compound of formula (I) is effected. Thus, for example, reaction of a compound of formula (III) with the R-absolute configuration with a compound of formula (IVA) with the R-absolute configuration would afford a compound of formula (I) with the R,R-absolute configuration.

A compound of formula (I) or a pharmaceutically acceptable salt thereof (hereinafter "the drug") may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or the like.

Typical carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides a method for treating obesity in humans or domestic mammals, which method comprises administering an effective, non-toxic amount of a compound of formula (I) or a pharmaceutically acceptable salt thereof to obese humans or domestic mammals.

In treating hyperglycaemic or obese humans the drug may be taken in doses, such as those described above, one to six times a day in a manner such that the total daily dose for a 70 kg adult will generally be

about 0.1 to 1000 mg, and more usually about 1 to 500 mg.

In treating hyperglycaemic or obese animals, especially dogs, the drug may be administered by mouth, usually once or twice a day and at about 0.025 mg/kg to 10 mg/kg, for example 0.1 mg/kg to 2 mg/kg.

The drug may be taken in doses such as those described above for treating obese humans.

The invention will now be illustrated with reference to the following Examples, which are not intended to limit the scope in any way. The Descriptions illustrate the preparation of intermediate compounds.

As used in the Examples, the term 'diastereoisomer' refers to a racemic pair of enantiomers.

Example 1

N-[2-(4-Sulphonamidophenyl)-1-methylethyl]-2-hydroxy-2-(3-trifluoromethylphenyl)ethanamine

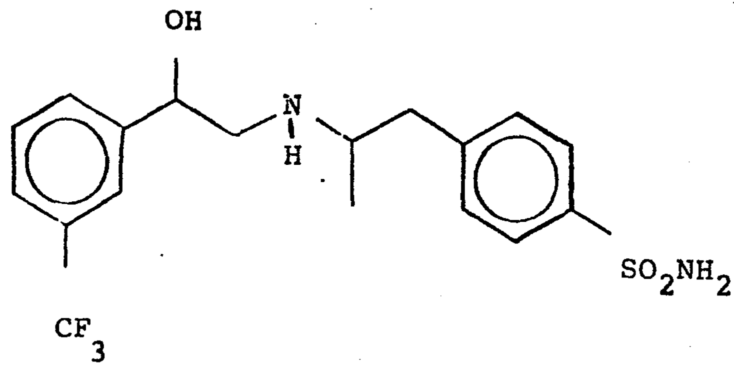

A mixture of 2-hydroxy-2-(3-trifluoromethylphenyl)ethanamine (4.3 g) and 1-(4-sulphonamidophenyl)propan-2-one (4.5 g) was dissolved in ethanol containing 3A molecular sieves and heated at reflux for 5 hours. The ethanol solution was decanted from the sieves, cooled in ice and sodium borohydride (0.79 g) added. After 15 minutes, the ethanol was evaporated, the residue dissolved in water and extracted with ethyl acetate. The organic extracts were dried (magnesium sulphate) and evaporated to give an oil which was purified (silica gel eluted with 8% methanol in chloroform). N-[2-(4-Sulphonamidophenyl)-1-methylethyl]-2-hydroxy-2-(3-trifluoromethylphenyl)ethanamine was crystallised from ethanol/water, mp 132-134°C as a 40:60 mixture of diastereoisomers.

$^1$H nmr; $\delta$ ($D_6$-DMSO) 9.1 (3H, d, J=6Hz), 7.0-7.6 (5H, m) 6.4-6.9 (2H, broad, replaceable by $D_2O$), 5.3 (1H, m), 2.5-3.1 (2H, broad, replaceable by $D_2O$), 2.7 (2H, d, J=6Hz), 2.2-2.6 (6H, m).

Example 2

N-[2-(4-N,N-Dimethylsulphonamidophenyl)-1-methylethyl]-2-hydroxy-2-(3-trifluoromethylphenyl)ethanamine

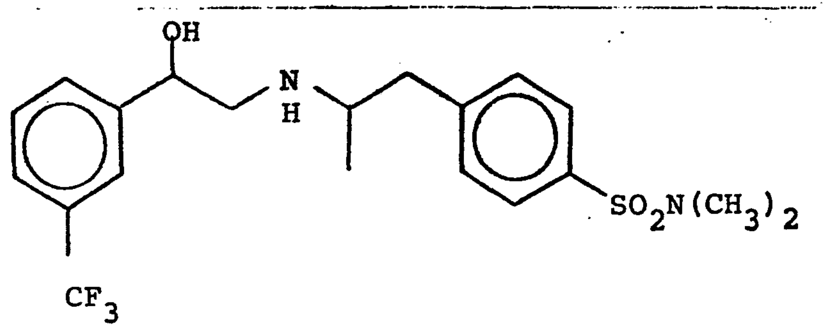

A mixture of 2-hydroxy-2-(3-trifluoromethylphenyl)ethanamine (2.04 g) and 1-(4-N,N-dimethylsulphonamidophenyl)propan-2-one (2.4 g) was dissolved in benzene and heated under reflux using a Dean and Stark apparatus for 4 hours. After cooling the benzene was evaporated, the residue dissolved in methanol and sodium borohydride (0.5 g) added at 0°C. After 30 minutes, the solvent was evaporated and the residue dissolved in water/ethyl acetate. The organic layer was separated, dried (magnesium sulphate) and evaporated to give an oil which was chromatographed on silica gel (eluted with 5% methanol-chloroform). N-[2-(4-N,N-Dimethylsulphonamidophenyl)-1-methylethyl]-2-hydroxy-2-(3-trifluoromethylphenyl)ethanamine was isolated as the monohydrochloride, mp 56-80°C as a 50:50 mixture of diastereoisomers.

$^1H$ nmr; $\tau$ $(CDCl_3)$ 8.7 (3H, d, J=6Hz), 7.3 (6H, s), 6.0-7.1 (5H, m), 5.2 (1H, broad, replaceable by $D_2O$), 4.4 (1H, m), 2.0-2.85 (8H,m), 1.1 (1H, broad, replaceable by $D_2O$), 0.0 (1H, broad, replaceable by $D_2O$).

0091749

Example 3

N-[2-(4-N,N-Dimethylsulphonamidophenyl)-1-methylethyl]-2- hydroxy-2-(3-chlorophenyl)ethanamine

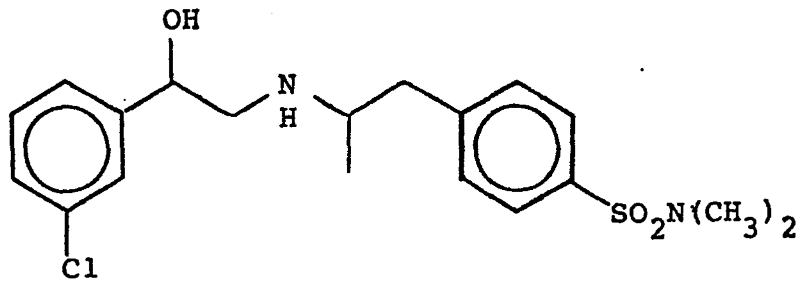

The compound was prepared as in Example 2 using 2-(3-chlorophenyl)-2-hydroxyethanamine instead of 2-hydroxy-2-(3-trifluoromethylphenyl)ethanamine and isolated as the hydrochloride salt, mp 63-69°C as a mixture of diastereoisomers.

$^1H$ nmr; $\tau$ ($CDCl_3$) 8.7 (3H, d, J=6Hz), 7.4 (6H, s), 6.2-7.2 (5H, m), 4.8 (1H, broad, replaceable by $D_2O$), 4.55 (1H,m)2.2-3.0 (8H, m), 1.2 (1H, broad, replaceable by $D_2O$), 0.1 (1H, broad, replaceable by $D_2O$).

Example 4

N-[2-(4-N,N-Di-n-propylsulphonamidophenyl)-1-methylethyl]-2-(3-chlorophenyl)-2-hydroxyethanamine

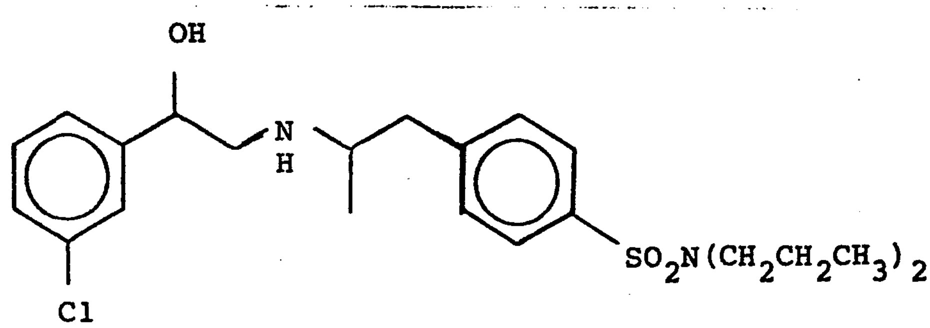

The compound was prepared as in Example 2 from 2-(3-chlorophenyl)-2-hydroxyethanamine (1.5 g) and 1-(4-N,N-di-n-propylsulphonamidophenyl)propan-2-one (2.5 g) and crystallised as the hydrobromide salt from ether, mp 151-165°C as a mixture of diastereoisomers.

$^1H$ nmr; $\tau$ ($CDCl_3$) 9.1 (6H, t, J=6Hz), 8.7 (3H, d, J=6Hz), 8.45 (4H, q, J=6Hz), 6.9 (4H, t, J=6Hz), 6.0-6.9 (5H, m), 4.45 (1H, m), 2.1-2.9 (8H, m), 1.9-4.0 (3H, broad, replaceable by $D_2O$).

Example 5

N-[2-(4-N,N-Di-n-propylsulphonamidophenyl)-1-methylethyl]-2-hydroxy-2-(3-trifluoromethyl-phenyl)ethanamine

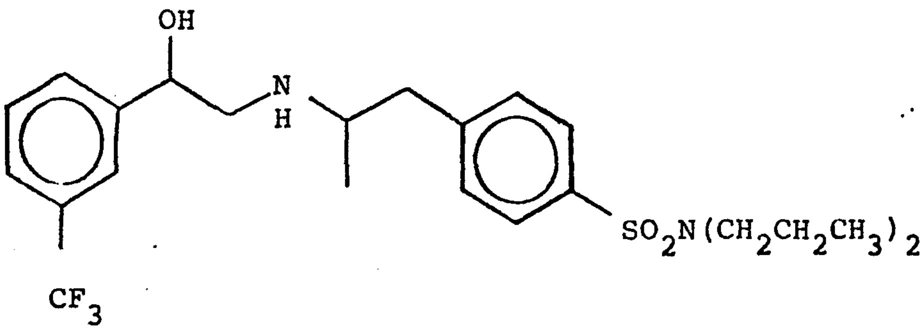

The compound was prepared as in Example 4 replacing 2-(3-chlorophenyl)-2-hydroxyethanamine by 2-hydroxy-2-(3-trifluoromethylphenyl)ethanamine and isolated as the hemifumarate, mp 58-64°C.

$^1H$ nmr; $\tau$ ($CDCl_3$) 9.15(6H, t, J=6Hz), 8.8 (3H, d), 8.5 (4H, q, J=6Hz), 6.9 (4H, t, J=6Hz), 6.2-7.3 (5H, m), 4.7 (1H, m), 3.2 (1H, s), 2.2-2.9 (8H, m), 1.3-2.1 (3H broad, replaceable by $D_2O$).

 

Example 6

N-[2-(4-(1-Piperidylsulphonyl)phenyl)-1-methylethyl]-2-hydroxy-2-(3-trifluoromethylphenyl)ethanamine

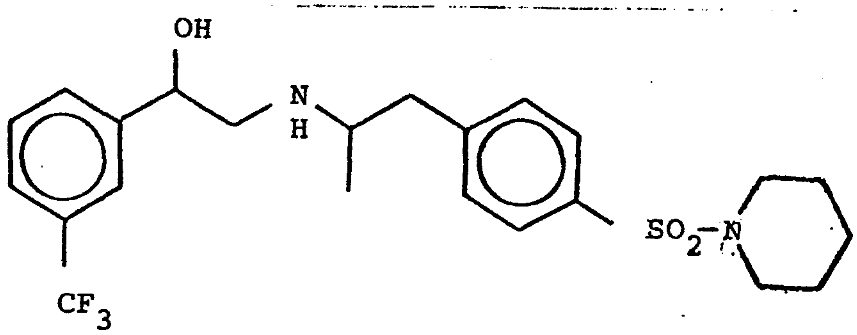

The compound was prepared as in Example 2 replacing 1-(4-N,N-dimethylsulphonamidophenyl)propan-2-one with 1-(4-(1-piperidylsulphonyl)phenyl)propan-2-one and crystallised as the hemifumarate, mp 76-90°C from diethylether as a 47:53 mixture of diastereoisomers.

$^1$H nmr; $\tau$ ($CDCl_3$) 8.8 (3H, d, J=6Hz), 8.1-8.8 (6H, m), 6.2-7.3 (9H, m), 4.8 (1H, m), 3.2 (1H, s), 2.1-2.7 (8H, m), 2.0 (3H, broad, replaceable by $D_2O$).

Example 7

N-[2-(4-N-n-Butylsulphonamidophenyl)-1-methylethyl]-2-(3-chlorophenyl)-2-hydroxyethanamine

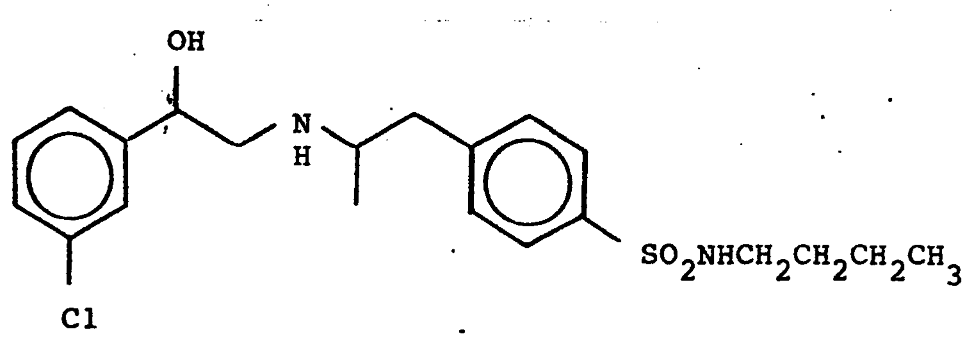

The compound was prepared as in Example 2 from 2-(3-chlorophenyl)-2-hydroxyethanamine (0.76 g) and 1-(4-N-n-butylsulphonamidophenyl)propan-2-one (1.2 g) and crystallised as the hemifumarate, mp 76-77°C as a 45:55 mixture of diastereoisomers.

$^1$H nmr; τ ($d_6$-DMSO) 9.1 (3H, t, J=6Hz), 8.85 (3H, d, J=6Hz), 8.4-8.7 (4H, m), 6.5-7.5 (7H, m), 5.1 (1H, m), 3.35 (1H, s), 2.0-3.1 (8H, m + 4H replaceable by $D_2O$).

Example 8

N-[2-(4-Benzylsulphonamidophenyl)-1-methylethyl]-2-(3-chlorophenyl)-2-hydroxyethanamine

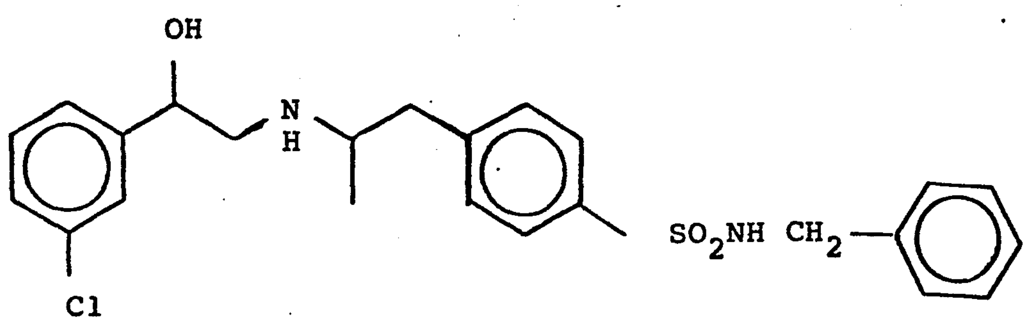

The compound was prepared as in Example 2 from 2-(3-chlorophenyl)-2-hydroxyethanamine (3.79 g) and 1-(4-N-benzylsulphonamidophenyl)propan-2-one (6.7 g) and crystallised from methanol as the monohydrate, mp 160-169°C as a mixture of diastereoisomers.

$^1H$ nmr; $\delta$ ($CDCl_3$) 9.0 (3H, d, J=6Hz), 6.9-7.6 (5H, m + 2H replaceable by $D_2O$), 6.0 (2H, s), 5.35 (1H, m), 4.5-5.6 (1H, br, replaceable with $D_2O$), 2.5-2.9 (11H, m), 2.25 (2H, d, J=8Hz), 2.0-3.2 (2H, broad, replaceable by $D_2O$).

Example 9

N-[2-(4-N-n-Butylsulphonamidophenyl)-1-methylethyl]-2-hydroxy-2-(3-trifluoromethylphenyl)ethanamine

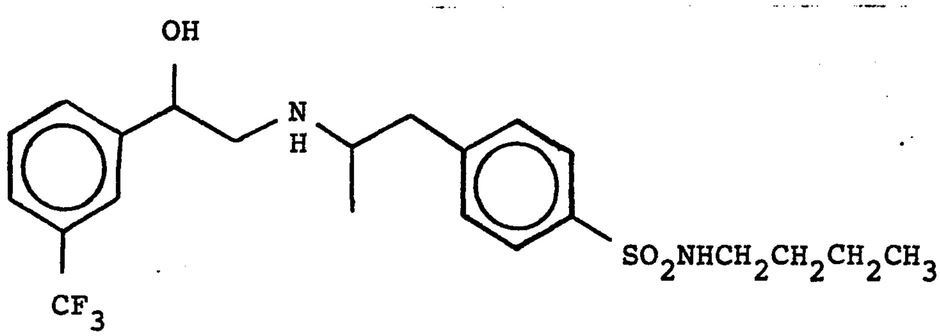

The compound was prepared as in Example 2 from 2-(3-trifluoromethylphenyl)-2-hydroxyethanamine (1.67g) and 1-(4-N-n-butylsulphonamidophenyl)propan-2-one (2.2g) and crystallised as the free base from benzene/hexane, m.p. 104-107$^{o}$C as a 47:53 mixture of diastereoisomers.

$^{1}$H nmr; $\tau$($CDCl_3$)

9.2 (3H,t), 8.92 (3H,d,J=6Hz), 8.4-8.9 (4H,m), 6.8-8.2 (7H,m + 3H replaceable by $D_2O$), 5.4 (1H,m), 2.1-2.8 (8H,m).

Example 10

N-[2-(4-N-Methylsulphonamidophenyl)-1-methylethyl]-2-hydroxy-2-(3-trifluoromethylphenyl)ethanamine

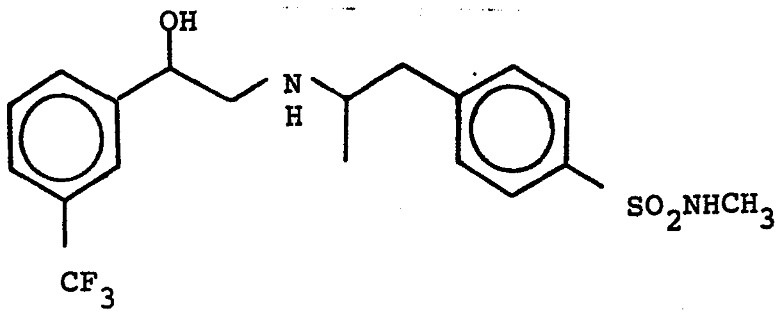

The compound was prepared as in Example 2 from 2-(3-trifluoromethylphenyl)-2-hydroxyethanamine (0.54g) and 1-(N-methylsulphonamidophenyl)propan-2-one (0.6g), and crystallised as the free base from benzene, m.p. 125-133°C, as a 52:48 mixture of diastereoisomers.

$^1$H nmr, $\tau$ ($d_6$-DMSO + $D_2O$):

9.00 (3H,d,J=6Hz), 7.5 (3H,s), 6.9-7.5 (5H,m), 5.25 (1H,m), 2.1-2.9 (8H,m).

Example 11

N-[2-(4-N,N-Diisopropylsulphonamidophenyl)-1-methylethyl]-2-hydroxy-2-(3-trifluoromethylphenyl)ethanamine

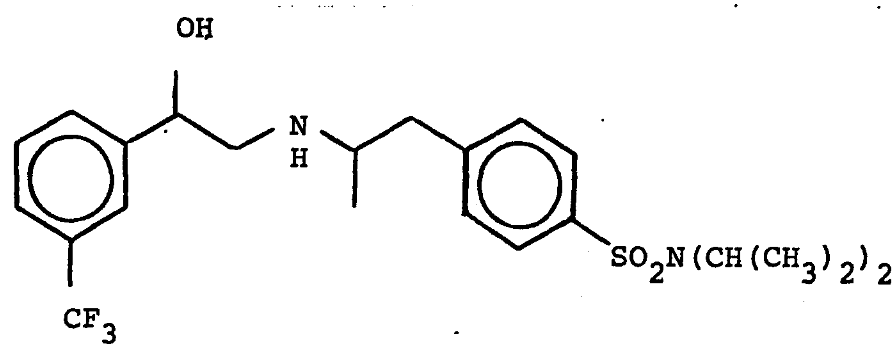

The compound was prepared as in Example 2 from 2-(3-trifluoromethylphenyl)-2-hydroxyethanamine (0.14g) and 1-(4-N,N-diisopropylsulphonamidophenyl)propan-2-one (0.20g), and crystallised as the hydrochloride salt from ether, m.p. 183-197$^{o}$C, as a 48:52 mixture of diastereoisomers.

$^{1}H$ nmr, $\tau$ ($d_6$-DMSO):

8.85 (15H,d,J=6Hz), 6.0-7.3 (7H,m), 4.8 (1H,m), 3.55 (1H, bd, replaceable by $D_2O$), 2.1-2.7 (8H,m), 0.0-1.3 (2H, broad, replaceable by $D_2O$).

Example 12

N-[2-(4-(4-Morpholinosulphonyl)phenyl)-1-methylethyl]-2-hydroxy-2-(3-trifluoromethylphenyl)ethanamine

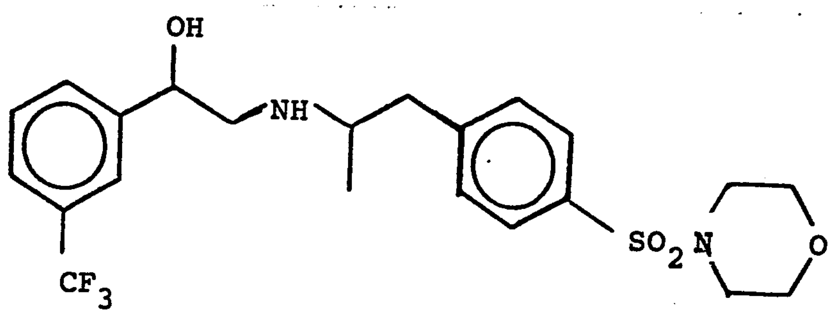

The compound was prepared as in Example 2 from 2-(3-trifluoromethylphenyl)-2-hydroxyethanamine (0.47g) and 1-[4-(4-morpholinosulphonyl)phenyl]propan-2-one (0.65g), and crystallised as the hemifumarate from methanol/ether, m.p. 182-187°C as a 29:71 mixture of diastereoisomers.

$^1$H nmr, $\tau$($d_6$-DMSo + $D_2$0):

9.05 (3H,d,J=6Hz), 6.7-7.4 (9H,m), 6.25-6.5 (4H,m), 5.15 (1H,m), 3.5 (1H,s), 2.2-2.7 (8H,m).

Example 13

N-[2-(4-N,N-Diethylsulphonamidophenyl)-1-methylethyl]-2-hydroxy-2-(3-trifluoromethylphenyl)ethanamine

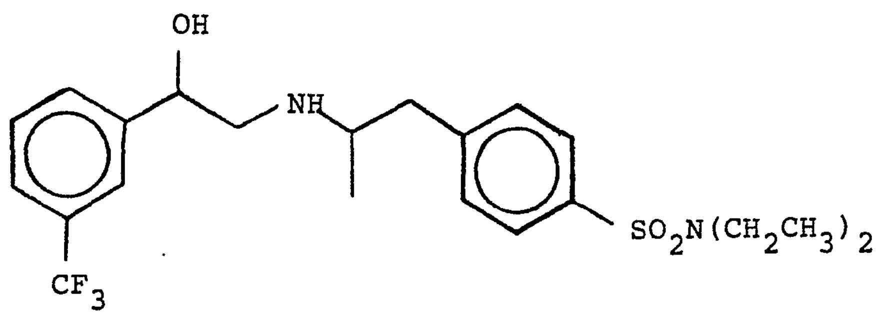

The compound was prepared as in Example 2 from 2-(3-trifluoromethylphenyl)-2-hydroxyethanamine (0.65g) and 1-(4-N,N-diethylsulphonamidophenyl)propan-2-one (0.85g) and isolated as the hemifumarate from methanol/ether, m.p. 153-156°C, as a 64:36 mixture of diastereoisomers.

$^1$H nmr, $\tau$($d_6$-DMSO):

9.0 (9H,t + d), 6.6-7.4 (9H,m), 5.15 (1H,m), 3.4 (1H,s), 2.8-3.8 (3H, broad, replaceable by $D_2O$), 2.1-2.7 (8H,m).

EXAMPLE 14

N-[2-(3-N,N-Diethylsulphonamidophenyl)-1-methylethyl]-2-hydroxy-2-(3-trifluoromethylphenyl)ethanamine

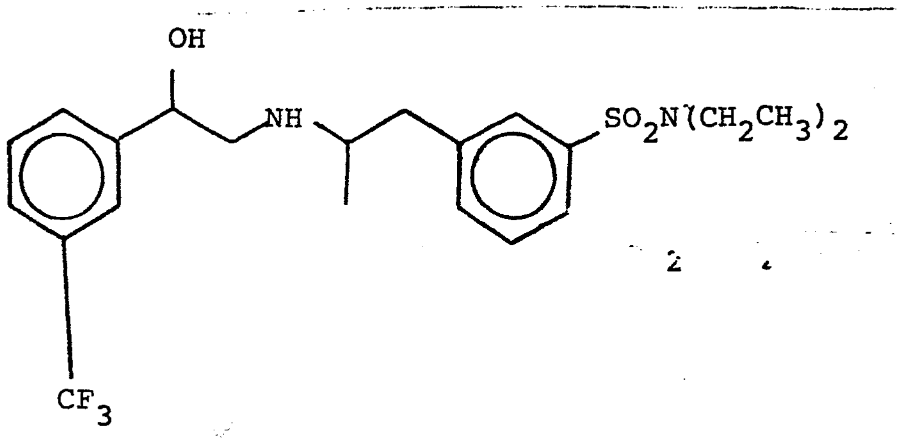

The compound was prepared as in Example 2 from 2-(3-trifluoromethylphenyl)-2-hydroxyethanamine (0.95g) and 1-(3-N,N-diethylsulphonamidophenyl)propan-2-one (1.25g), and isolated as the hydrochloride salt from methanol / ether m.p. 135-149$^{o}$C, as a 60:40 mixture of diastereoisomers.

$^{1}$H nmr, $\tau$($d_6$-DMSO):

8.8-9.1 (9H,t + d), 6.3-7.5 (9H,m), 4.8 (1H,m), 3.5 (1H, bd, replaceable by $D_2O$), 2.0-2.7 (8H,m), -0.3-1.2 (2H, broad, replaceable by $D_2O$).

Description 1

1-(4-Sulphonamidophenyl)propan-2-one

To an ice cooled stirred solution of chlorosulphonic acid (125 ml) was added dropwise phenylacetone (30 g) keeping the temperature below 5<C. After the complete addition, the mixture was stirred for a further 30 minutes below 5<C and then for a further 2 hours whilst the temperature rose to ambient. The mixture was poured gently onto chipped ice and extracted with chloroform after the ice had melted. The organic extracts were dried (magnesium sulphate) and evaporated to give a brown oil. 0.880 Ammonia solution (100 ml) was then added to the oil and the mixture heated on a steam bath for 20 minutes. A dark oily layer was formed and removed by decanting. On cooling the aqueous layer, crystalline sulphonamide was formed. This was recrystallised from methanol/water, mp 122-125<C, to give 1-(4-sulphonamidophenyl)propan-2-one.

$^1H$ nmr; $\tau$ ($d_6$ DMSO-$CDCl_3$) 7.78 (3H, s), 6.15 (2H, s), 2.6 (2H, d, J=8Hz), 2.5-3.0 (2H, broad replaceable by $D_2O$), 2.1 (2H, d, J=8Hz).

Description 2

1-(4-N,N-Dimethylsulphonamidophenyl)propan-2-one

The compound was prepared as in Description 1 replacing 0.880 ammonia solution with a solution of dimethylamine in methanol.

$^1H$ nmr; $\tau$ ($CDCl_3$) 7.8 (3H, s), 7.25 (6H, s), 6.1 (2H, s), 2.7 (2H, d, J=8Hz), 2.3 (2H, d, J=8Hz).

Description 3

1-(4-N,N-Di-n-propylsulphonamidophenyl)propan-2-one

The compound was prepared as in Description 1 replacing 0.880 ammonia solution with di-n-propylamine.

$^1H$ nmr $\tau$ ($CDCl_3$) 9.1 (6H, t, J=8Hz), 8.45 (4H, m ), 7.78 (3H, s), 6.9 (4H, t, J=8Hz), 6.2 (2H, s), 2.6 (2H, d, J=8Hz), 2.2 (2H, d, J=8Hz).

Description 4

1-(4-(1-Piperidylsulphonyl)phenyl)propan-2-one

The compound was prepared as in Description 1 replacing 0.880 ammonia solution with piperidine.

$^1H$ nmr; $\tau$ ($CDCl_3$) 8.0-8.8 (6H, m), 7.8 (3H, s), 6.8-7.3 (4H, m), 6.2 (2H, s), 2.7 (2H, d, J=8Hz), 2.3 (2H, d, J=8Hz).

Description 5

1-(4-N-n-Butylsulphonamidophenyl)propan-2-one

The compound was prepared as in Description 1 replacing 0.880 ammonia solution with n-butylamine.

$^1H$ nmr; τ ($CDCl_3$) 9.2 (3H, t), 8.5-9.0 (4H, m), 7.8 (3H, s), 7.1 (2H, t), 6.2 (2H, s), 4.8 (1H, t, replaceable by $D_2O$), 2.8 (2H, d, J=8Hz), 2.2 (2H, d, J=8Hz).

DESCRIPTION 6

1-(4-Chlorosulphonylphenyl)propan-2-one

To an ice-cooled, mechanically stirred solution of chlorosulphonic acid (125ml), was added phenylacetone (31g) always maintaining the temperature below 5°C. After the complete addition (approx. 2 hours), the mixture was stirred for an additional 1 hour at <5°C, then allowed to warm to ambient temperature over 2 hours. The dark brown mixture was added very slowly, with stirring, onto chipped ice to give a pale yellow solid. Column chromatography on silica gel eluting with hexane/ether (50:50) gave a minor product, 1-(3-chlorosulphonylphenyl)propan-2-one (Rf, $SiO_2$/ether, 0.6) m.p. 66-69 (ether/40-60 petrol).

$^1H$ nmr, $\tau$ ($CDCl_3$):

7.7 (3H,s), 6.1 (2H,s), 2.35-2.45 (2H,m), 1.8-2.15 (2H,m).

The major product was the title compound, 1-(4-chlorosulphonylphenyl)propan-2-one, (Rf, $SiO_2$/ether, 0.5), m.p. 96-98° (hexane/ether).

$^1H$ nmr, $\tau$ ($CDCl_3$):

7.7 (3H,s), 6.08 (2H,s), 2.5 (2H,d,J=8Hz), 1.95 (2H,d,J=8Hz).

DESCRIPTION 7

1-(4-N-Methylsulphonamidophenyl)propan-2-one

To a solution of 1-(4-chlorosulphonylphenyl)propan-2-one (1g) in pyridine (10ml) was added a solution of methylamine (about 33% in ethanol) (5ml) and the mixture left to stand at ambient temperature for 1 hour. The mixture was poured into water (300ml) and extracted with ethyl acetate. The organic layer was washed with 2M HCl, dried ($MgSO_4$) and evaporated to give the compound as a pale yellow solid, m.p. 100-105°C.

$^1H$ nmr, $\tau$ ($CDCl_3$):

7.75 (3H,s), 7.3 (3H,d, collapses to a singlet on $D_2O$ addition), 6.2 (2H,s), 5.2 (1H,m, replaceable by $D_2O$), 2.6 (2H,d,J=8Hz), 2.1 (2H,d,J=8Hz).

DESCRIPTION 8

1-(4-(4-Morpholinosulphonyl)phenyl)propan-2-one

The compound was prepared as in Description 7 replacing methylamine with morpholine.

$^{1}H$ nmr, $\tau(CDCl_3)$:

7.75 (3H,s), 6.95 (4H,m), 6.2 (4H,m), 6.15 (2H,s), 2.55 (2H,d,J=8Hz), 2.2 (2H,d,J=8Hz).

DESCRIPTION 9.

1-(4-N,N-Diethylsulphonamidophenyl)propan-2-one

The compound was prepared as in Description 7 replacing methylamine with diethylamine, to give an oil which crystallised on standing, m.p. 66-64$^{\circ}$C.

$^{1}$H nmr, $\tau$($CDCl_3$):

8.98 (6H,t,J=8Hz), 7.8 (3H,s), 6.75 (4H,q,J=8Hz), 6.22 (2H,s), 2.68 (2H,d,J=8Hz), 2.2 (2H,d,J=8Hz).

DESCRIPTION 10

1-(4-N,N-Diisopropylsulphonamidophenyl)propan-2-one

The compound was prepared as in Description 7 replacing methylamine with diisopropylamine.

$^1$H nmr, $\tau$($CDCl_3$):

8.75 (12H,d), 7.82 (3H,s), 6.3 (2H,m), 6.24 (2H,s), 2.7 (2H,d,J=8Hz), 2.18 (2H,d,J=8Hz).

DESCRIPTION 11

1-(3-N,N-Diethylsulphonamidophenyl)propan-2-one

The compound was prepared as in Description 7 replacing 1-(4-chlorosulphonylphenyl)propan-2-one with 1-(3-chlorosulphonylphenyl)propan-2-one, and methylamine with diethylamine.

$^1$H nmr, $\tau$($CDCl_3$):

8.85 (6H,t), 7.78 (3H,s), 6.7 (4H,q), 6.2 (2H,s), 2.45-2.7 (2H,m), 2.0-2.4 (2H,m).

Demonstration of Effectiveness of Compounds

(i) Effect on energy expenditure

The effect of the compounds on the energy expenditure of mice was demonstrated by means of the following procedure:

Female CFLP mice each weighing approximately 24 g, were given food and water ad lib before and during the experiment. The compounds were dissolved in water by addition of one mole of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content, following the principles described by J.B. de V. Weir, J. Physiol. (London) 109, 1-9 (1949). The results (Table 1) are expressed as a percentage of the rate of energy expenditure of the mice dosed with water.

| COMPOUND OF EXAMPLE NO | DOSE (mg/kg po) | MEAN ENERGY EXPENDITURE % (0-3h) | (0-21h) |
|---|---|---|---|
| 1 | 22.4 | 146 | 109 |
| 2 | 25.9 | 143 | 112 |
| 3 | 24.1 | 138 | 111 |
| 4 | 29.6 | 156 | 112 |
| 5 | 30.3 | 146 | 112 |
| 6 | 29.4 | 152 | 110 |
| 7 | 26.9 | 180 | 125 |
| 8 | 26.5 | 150 | 114 |
| 9 | 22.9 | 153 | 115 |
| 10 | 20.7 | 145 | 105 |
| 12 | 26.5 | 131 | 105 |
| 13 | 25.8 | 152 | 117 |

(ii) Anti-obesity Activity

The compounds were administered by oral gavage in water or carboxylmethyl-cellulose suspension to genetically obese mice daily for 28 days. At the end the carcass composition was determined. The results obtained were as follows:-

| COMPOUND OF EXAMPLE NO. | DOSE mg/kg p.o. | g LIPID/MOUSE Treated | Control |
|---|---|---|---|
| 2 | 13.0 | 21.93 | 26.24 |
| 3 | 12.1 | 18.36 | 23.25 |
| 5 | 15.2 | 22.01 | 22.99 |
| 8 | 13.3 | 20.82 | 22.78 |

(iii) Anti-hyperglycaemic activity

Female CFLP mice, weighing approximately 25 g, were fasted for 24 hours prior to the study. The compounds under study were administered orally as an aqueous solution to each of 6 mice. 30 Minutes later a blood sample (20 µl) was obtained from the tail for the analysis of blood glucose. Immediately after taking this blood sample, glucose (1 g/kg body weight) was administered subcutaneously to each mouse. 6 Mice were given water as a control. Blood samples were then obtained from each mouse at 30 minute intervals for 120 minutes.

Compounds that produced a significant (P 0.05) reduction of blood glucose, compared with control mice given water, at any time interval, were considered active. The area under the blood glucose curve over the 2 hour period after the administration of the glucose was calculated for each compound and compared with the value for control animals.

| COMPOUND OF EXAMPLE NO. | DOSE (μmol/kg) | % REDUCTION IN AREA UNDER BLOOD GLUCOSE CURVE |
|---|---|---|
| 3 | 12.5 | 64 |
| 11 | 100 | 26 |
| 14 | 100 | 34 |

(iv) Conscious Rat Model

The pressure pulse from the rat's tail blood flow is recorded via a sensor round the tail and displayed on a storage oscilloscope. Heart rate is obtained by counting the number of pulses over a fixed time interval (5 secs). To ensure adequate peripheral blood flow, the rats are kept in an incubator at 35°C for 30 mins before each recording. To minimise stress and thus ensure as low a basal heart rate as possible, the rats are trained in the procedure prior to the actual experiment. After an initial reading, the rats are dosed orally, then further readings taken every 45 minutes. Results are expressed as the mean net change in heart rate of drug-treated groups after subtracting changes in the control group.

| Compound of Example No. | Dose (mg/kg po) | Increase in heart rate (beats per min.) | |
|---|---|---|---|
| | | 45 mins. | 90 mins. |
| 1 | 11.4 | 105 | 115 |
| 4 | 14.9 | 67 | 65 |
| 5 | 15.2 | 1 | 15 |
| 7 | 13.4 | 60 | 116 |
| 9 | 4.6 | 34 | 34 |
| 10 | 4.2 | 65 | 70 |
| 11 | 5.2 | 11 | 10 |
| 12 | 5.3 | 19 | 11 |

CLAIMS

0091749

1. A compound of formula (I):

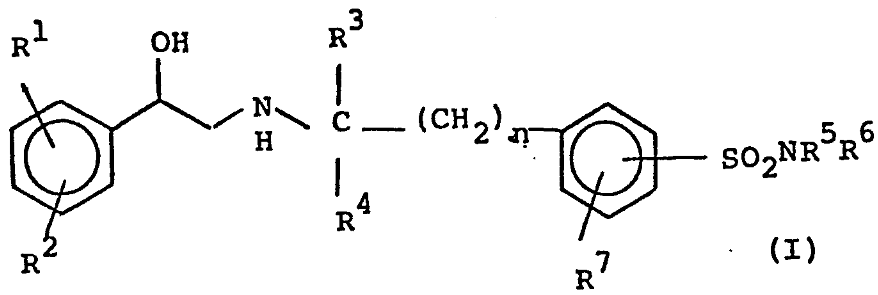

(I)

or a salt thereof, wherein;

$R^1$ is hydrogen, halogen or trifluoromethyl,

$R^2$ is hydrogen or halogen,

$R^3$ is hydrogen or methyl,

$R^4$ is hydrogen or methyl,

$R^5$ is hydrogen, $C_{1-10}$ straight or branched alkyl or optionally substituted benzyl,

$R^6$ is hydrogen, $C_{1-10}$ straight or branched alkyl, or optionally substituted benzyl,

or $R^5$ and $R^6$ taken together with the attached nitrogen atom represent a heterocylic ring,

$R^7$ is hydrogen or halogen, and

n is 1 or 2

2. A compound according to claim 1, in which $R^3$ is hydrogen and $R^4$ is simultaneously methyl.

3. A compound according to claim 1 or 2, in which $R^5$ or $R^6$ is a $C_{1-5}$ straight chain alkyl group.

4. A compound according to claim 1 or 2, in which $R^5$ and $R^6$, together with the attached nitrogen atom, represent a five or six membered heterocyclic ring optionally containing an additional hetero-atom selected from oxygen, sulphur or nitrogen.

5. A compound according to claim 1, selected from:

N-[2-(4-sulphonamidophenyl)-1-methylethyl]-2-hydroxy-2-(3-trifluoromethylphenyl)ethanamine;

N-[2-(4-N,N-dimethylsulphonamidophenyl)-1-methylethyl]-2-hydroxy-2-(3-trifluoromethylphenyl)ethanamine;

N-[2-(4-N,N-dimethylsulphonamidophenyl)-1-methylethyl]-2-hydroxy-2-(3-chlorophenyl)ethanamine;

N-[2-(4-N,N-di-n-propylsulphonamidophenyl)-1-methylethyl]-2-(3-chlorophenyl)-2-hydroxyethanamine;

N-[2-(4-N,N-di-n-propylsulphonamidophenyl)-1-methylethyl]-2-hydroxy-2-(3-trifluoromethylphenyl)ethanamine;

N-[2-(4-(1-piperidylsulphonyl)phenyl)-1-methylethyl]-2-hydroxy-2-(3-trifluoromethylphenyl)ethanamine;

N-[2-(4-N-n-butylsulphonamidophenyl)-1-methylethyl]-2-(3-chlorophenyl)-2-hydroxyethanamine;

N-[2-(4-N-benzylsulphonamidophenyl)-1-methylethyl]-2-(3-chlorophenyl)-2-hydroxyethanamine;

N-[2-(4-N-n-butylsulphonamidophenyl)-1-methylethyl]-2-hydroxy-2-(3-trifluoromethylphenyl)ethanamine;

N-[2-(4-N-methylsulphonamidophenyl)-1-methylethyl]-2-hydroxy-2-(3-trifluoromethylphenyl)ethanamine;

N-[2-(4-N,N-diisopropylsulphonamidophenyl)-1-methylethyl]-2-hydroxy-2-(3-trifluoromethylphenyl)ethanamine;

N-[2-(4-(4-morpholinosulphonyl)phenyl)-1-methylethyl]-2-hydroxy-2-(3-trifluoromethylphenyl)ethanamine;

N-[2-(4-N,N-diethylsulphonamidophenyl)-1-methylethyl]-2-hydroxy-2-(3-trifluoromethylphenyl)ethanamine; and

N-[2-(3-N,N-diethylsulphonamidophenyl)-1-methylethyl]-2-hydroxy-2-(trifluoromethylphenyl) ethanamine.

N-[2-(4-N,N-diethylsulphonamidophenyl)-1-methylethyl]-2-hydroxy-2-(3-trifluoromethylphenyl)ethanamine; and

N-[2-(3-N,N-diethylsulphonamidophenyl)-1-methylethyl]-2-hydroxy-2-(trifluoromethylphenyl) ethanamine.

6. A process for producing a compound of formula (I) as defined in claim 1, or a salt thereof, which comprises reducing (a) an oxo group and/or double bond and/or cleaving a benzyl group of a compound of formula (II):

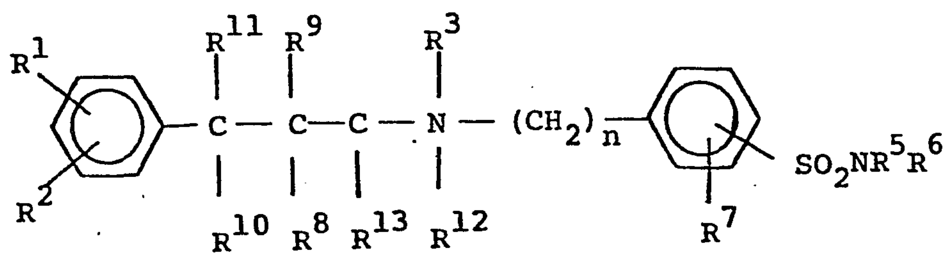

(II)

wherein $R^1$, $R^2$, $R^3$, $R^5$, $R^6$, $R^7$ and n are as defined in relation to formula (I),
$R^8$ is hydrogen or together with $R^{13}$ forms an oxo-group or together with $R^7$ forms a bond,
$R^9$ is hydrogen or together with $R^8$ forms an oxo-group,
$R^{10}$ is hydroxyl or together with $R^{11}$ forms an oxo-group,
$R^{11}$ is hydrogen or together with $R^{10}$ forms an oxo-group,
$R^{12}$ is a group $R^4$ as defined in relation to formula (I) or together with $R^{13}$ forms a bond,

$R^{13}$ is hydrogen or benzyl or together with $R^{12}$ or $R^8$ forms a bond, provided that there is no more than one oxo-group represented by any of $R^8$ to $R^{11}$; and optionally thereafter forming a salt of the compound of formula (I) so formed,

or (b) reacting a compound of formula (III):

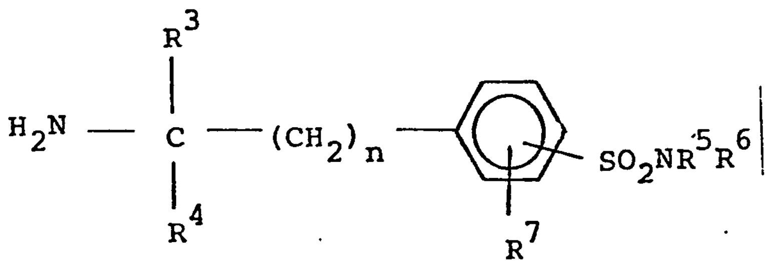

(III)

wherein $R^3$, $R^4$, $R^5$, $R^6$, $R^7$ and n are as defined in relation to formula (I);

with a compound of formula (IV):

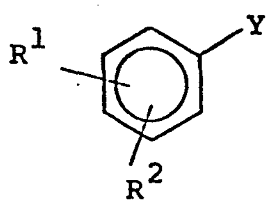

(IV)

wherein $R^1$ and $R^2$ are as defined in relation to formula (I), and Y is a group capable of reacting with the amine of formula (III) thus forming a compound of formula (I), and optionally thereafter forming a salt of the compound of formula (I) so formed,

or (c) reacting a compound of formula (V):

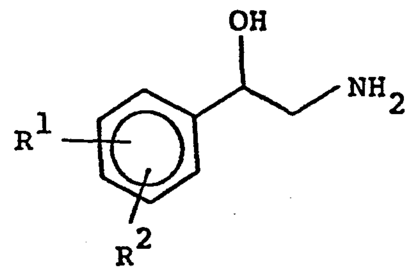

(V)

wherein $R^1$ and $R^2$ are as defined in relation to formula (I);

with a compound of formula (VI):

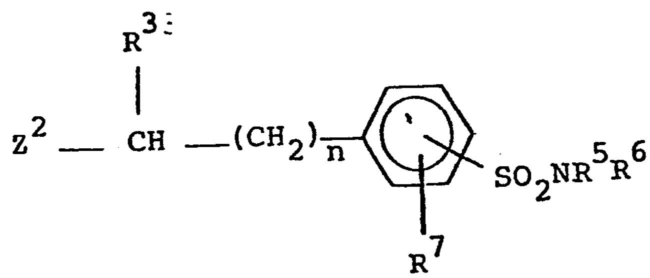

(VI)

wherein $R^3$, $R^5$, $R^6$, $R^7$ and n are as defined in relation to formula (I) and $Z^2$ is a leaving group, and optionally thereafter forming a salt of the compound of formula (I) so formed.

7. A pharmaceutical composition comprising a compound according to any one of claims 1 to 5 and a pharmaceutically acceptable carrier therefor.

8. A composition according to claim 7 in unit dosage form.

9. A compound according to any one of claims 1 to 5 for use in treating the human or animal body.